Europäisches Patentamt

European Patent Office

Office européen des brevets

(19)

(11) Publication number: **0 553 054 A2**

# EUROPEAN PATENT APPLICATION

(12)

(21) Application number: **93610005.6**

(22) Date of filing: **22.01.93**

(51) Int. Cl.5: **G01N 35/00**, G01N 30/14, G01N 30/46

(30) Priority: **22.01.92 DK 80/92**

(43) Date of publication of application:
**28.07.93 Bulletin 93/30**

(84) Designated Contracting States:
**DE DK ES FR GB IE NL SE**

(71) Applicant: **Slagteriernes Forskningsinstitut**
**Maglegaardsvej 2**
**DK-4000 Roskilde(DK)**

(72) Inventor: **Hansen-Moller, Jens**
**Nygade 23**
**DK-4840 NR. ALSLEV(DK)**

(74) Representative: **Rotne, Jens Styrup et al**
**c/o Internationalt Patent-Bureau Hoeje**
**Taastrup Boulevard 23**
**DK-2630 Taastrup (DK)**

(54) **A process for the examination of individual items for the contents of a fat-soluble compound.**

(57) A sample from individual items may after extraction be analyzed for the contents of one or more fat-soluble compound(s) by HPLC chromatography, the separation column during injection of the analysis sample being protected by means of a precolumn which during the further course of analysis is switched out of serial connection with the separation column so that it may be purified by flushing with appropriate fat-soluble agents. The apparatus for carrying out the method is composed of standard HPLC components. The method may be used for detecting or sorting-out such individual items for which the analysis results show excess of a predetermined threshold value of the fat-soluble compound(s) searched for.

FIG.3

The invention relates to a method of examining inidividual items for the contents of at least one fat-soluble compound, an apparatus for use in automatically carrying out the method and use of said method or apparatus for detecting or sorting individual items on the basis of the contents of at least one fat-soluble compound.

The method is in particular developed for the examining of individual pieces of meat in the presence of substances connected with male pig smell.

Pieces of meat from uncastrated male pigs or boars may during boiling or roasting emit an unpleasant aroma, so-called male pig smell or boar taint, whereas such smell incoveniences seldom occur when cooking meat from castrated male pigs. In order to avoid such smell nuisances male pigs are therefore frequently subjected to castration at an early age. This implicates, however, that the feeding efficiency is lowered and the disease frequency rises and at the same time the meat percentage in carcasses decreases.

Methods have been suggested of detecting whether individual carcasses will develop boar taint during cooking cut pieces therefrom to make it possible to sort out such carcasses prior to the further processing into cuts determined for retail distribution and to use the discarded carcasses in industry, for example as canned food or in the production of sausages, the smell emission being in this respect of no importance.

Such methods are based on determining on an extract of a sample from the individual carcasses the analytic data for compounds contained therein which are connected with boar taint, e.g. skatole, indole, androstenone or other substances, and comparing such data to preproduced standards. In this connection DK patent No. 155 200 discloses the reaction of the extract with a colour developing agent of compounds contained therein and connected with boar taint and subsequent spectrophotometric determination, DK patent No. 145 536 deals with the use of IR-spectrophometric transmission data and DK patent No. 154 667 relates to the use of spectrofluorometric data.

J.A. Garcia-Regueiro et. al., J. High Res. Chrom. & Chrom. Comm., 9, 1986, 362 to 3, states that 5α-androst-16-en-3-one (androstenone) and skatole are compounds connected with male pig smell and describes a method of determining skatole and indole in samples of back fat from pigs, thereby effecting after purification of an extract of a fat sample a HPLC-analysis on an analysis sample of the purified extract by injecting the sample into a mobile phase passed through a separation column provided with a detector, the signals of which are converted to an expression of the contents of the compound.

Besides freezing, filtering, evaporation and resolving the mentioned extract purification involves a passage through a column filled with Florisil. Since such a column has to be regularly replaced and in spite of apparently good results the method is hardly appropriate for carrying out a long series of analyses.

It has been tried to use sep-pak minicolumns in the purification but they have been discarded for giving less reproducible results than columns with Florosil.

J.A. Garcia-Regueiro et. al., Proceedings I, 32nd European Meeting of Meat Research Workers, Gent, 1986, states, moreover, that androstenone may be determined spectrophometrically in a purified extract from a fat sample after derivatization by 2.4-dinitrophenyl hydrazine. The purification of the extract was substantially effected as stated above. The derivatization took 30 minutes at 60°C, following which the sample was again purified prior to the HPLC or HRGC analysis.

A determination of skatole and optionally androstenone in blood samples instead of in fat samples might appear to be promising, since the purification of such a blood sample substantially merely involves the precipitation by means of an excess of acetonitrile and a following separation of fibrinogene. However, a HPLC analysis of such a prepared extract of a blood sample results in a bad separation of the peaks in the registered chromatogram owing to a strongly reduced efficiency of the HPLC system.

It is the object of the invention to provide a method appropriate for long series of analyses and thus industrially applicable for detecting or sorting individual items from the results of the analysis, such as sorting out slaughtered, uncastrated male pigs which during cooking might emit a deviating smell.

It has now surprisingly turned out that in spite of Garcia-Regueiro's rejection of purification by means of minicolumns and in spite of the fact that precolumns according to known HPLC technique are generally used for concentration of a sample or for protection of a column against particles originating from injection equipments and the like, it is possible to make use of a precolumn to delay fat substances in an analysis sample and thus protect the separation column proper against occlusion therewith and that the precolumn may be substantially regenerated while the analysis sample purified of fat substances is passing the separation column.

The method concerned which as regards each item individual comprises the repetition of the following series of operations:

taking a sample from the individual,

treating the sample with a solvent for the fat-soluble compound,

separation of solid particles from the obtained solution, and

carrying out the HPLC analysis on an analysis sample of the purified solution, thereby causing the sample to be injected into a mobile phase passed through a separation column provided with a detector the signals of which are automatically converted to an expression of the contents of the compound, is accordingly in a first aspect characterized in that the analysis sample in the mobile phase is passed through a precolumn positioned in front of the separation column and that the precolumn is subsequently washed separately for a predetermined period of time.

In this manner a large number of samples may be analyzed during a relatively short time and the column may at the same time be used for a longer period of time without replacement.

The washing of the precolumn is preferably effected while reversing the flow direction therein, the analysis sample in the mobile phase being preferably passed further through the separation column while washing the precolumn.

This embodiment is further advantageous in analysis of samples with a residual content of solid particles, e.g. plasma or serum containing fibrinogene.

The apparatus for automatically carrying out this method includes a separation column and pumping means for passing a mobile phase through the column and a device arranged in front of the column for injecting an analysis sample into the mobile phase and is characterized in that it includes a precolumn arranged between the means for injecting an analysis sample and the separation column, and that valves are provided for coupling the precolumn into a separate wash flow for a predetermined time without changing the direction of flow in the separation column.

A preferred embodiment of this apparatus is characterized in that the valves reverse the direction of flow in the precolumn when coupled in a separate wash flow for a predetermined time.

A preferred embodiment of the method concerned is characterized by the following steps:

that the mobile phase is passed through the precolumn and the separation column,

that an analysis sample representing a single individual is automatically removed from a storage of separately produced and stored extracts,

that a predetermined amount of the sample is injected into the mobile phase and passed along with it through the precolumn,

that the analysis sample in the mobile phase is passed further on in the separation column while reversing the direction of flow in the precolumn and washing the precolumn with one or more, preferably fat-soluble flush phases and - if desired - finally with the same agent as the mobile phase, and

that the flow direction in the precolumn is subsequently reversed again.

A preferred embodiment of the present apparatus is characterized in that it includes an autosampler with an injection valve for an analysis sample, two six-port switching valves either of which may occupy two positions, a first pump for pumping a mobile phase from a reservoir therefor through a selected port in one of the six-port switching valves, a second pump which according to choice may pump a mobile phase, a supplementary mobile phase and flush phases from reservoirs therefor through a second selected port in one of the six-port switching valves, a precolumn delaying the fat substance, a separation column, a detector and pipe connections between the parts whereby the pipe connections are coupled in such a manner that the precolumn in a first combination of valve positions forms part of the following first series connection:

first pump, autosampler, precolumn with non-inverted flow, separation column and detector,

in a second combination of valve positions the precolumn forms part of the second series connection:

second pump, precolumn with reverse flow, autosampler and outlet, and

in a third combination of valve positions the precolumn forms part of the third series connection:

second pump, autosampler, precolumn with non-inverted flow and outlet.

It has further been found that androstenone which is an example of a non-fluorescent compound that is considered to be connected with male pig smell, may be derivatizised far more rapidly and at a lower temperature than stated by Garcia-Regueiro. When using a catalyst for the derivatization reaction said reaction is accomplished at ambient temperature within 2 to 4 minutes.

Accordingly, the present method as stated above is in a second aspect characterized by adding a derivatization agent which will make the non-fluorescent compound fluorescent together with a catalyst for the derivatization reaction to the solution or analysis sample, for the determination by means of a fluorescence detector of a non-fluorescent, fat-soluble compound, optionally together with one or more other fat-soluble compounds that are fluorescent.

The derivatization agent for androstenone is preferably selected among dansylhydrazine, 4'-hydrazino -2-stilbazole, 4'-hydrazino-2-oxo-1,3-diazole and 2,4-dinitrophenyl hydrazine, and is in particular dansyl hydrazine.

As catalysts for the derivatization reaction Lewis-acids, in particular boron trifluoride, have turned out to be suitable.

The present method may in this second aspect advantageously be carried out as stated above in connection with the first aspect of the method.

As stated above, a HPLC analysis of an analysis sample based on a blood sample from which plasma proteins are removed by precipitation with an excess of acetonitrile, results in a bad separation of the peaks in the registered chromatogram.

It is further observed that if the solvent used in the extraction of a fatty sample and the mobile phase chromatographs substantially differently, there is an upper limit of the volume of the analysis sample injected into the mobile phase, above which a peak broadening occurs in the registered chromatogram and a corresponding reduction of the selectivity for the performed analysis.

It has turned out that said inconveniences may be eliminated or substantially diminished if the analysis sample prior to passing the separation column is diluted with a selected, supplementary mobile phase.

Accordingly, the present method in a third aspect is characterized in that the mobile phase is further passing a mixing chamber positioned in front of the column and while the injected amount of analysis sample is passing the mixing chamber through an opening therein a further mobile phase is being admixed therein.

If the sample is a blood sample from an animal it is advantageous to add acetonitrile in a ratio of 1 to 3 parts by volume of acetonitrile to 1 part by volume of the sample to plasma or serum from the sample, for the precipitation of plasma proteins prior to removing solid particles, and while the analysis sample is passing the mixing chamber so much supplementary mobile phase is being admixed that the acetonitrile content of the sample is reduced to 45 % by volume or less.

The present method may in its third aspect frequently advantageously be carried out as explained above in connection with the first aspect of the method and/or if for example a determination of androstenone is desired, also as described above in connection with the second aspect of the method.

An apparatus for that purpose is characterized in that it further comprises a mixing chamber with three openings, the first one of which is connected through pipe with the injection valve of the autosampler and the second one through pipes with the precolumn, and that the precolumn of a first and second combination of valve positions forms part of the first and second series connection, respectively, as mentioned above, in that the third opening of the mixing chamber is blocked, and that the precolumn in a third combination of valve positions forms part of the first series connection as mentioned above, the third opening of the mixing chamber being at the same time connected with the second pump.

The detection principle for the detector is preferably fluorescence.

As a control of the performed analyses it may be advantageous that the analysis sample contains a known amount of a reference compound related to one of the compounds for analysis. Accordingly, an embodiment of the present method is characterized in that a predetermined amount of a reference compound is added to the fatty sample prior to or during the extraction thereof, said compound being related to the fat-soluble compound to be detected by the HPLC analysis, preferably 2-, 4-, 5-, 6- or 7-methylindole for the determination of skatole, and androstanone for the determination of androstenone.

In carrying out the present method it may be advantageous in connection with the determination of further fat-soluble compounds by the HPLC analysis to change the mobile phase and/or change the flow rate of the mobile phase to a faster pass-through after the analysis sample has been injected into the separation column. In this manner the time of analyzing may be shortened without deteriorating the exactitude.

Instead of an above mentioned precolumn derivatization a post column derivatization may be performed, i.e. by reacting an eluate from the separation column with a derivatization agent and subsequent detection of a derivative in the detector.

Preferred extraction agents for a fatty sample is selected among methanol, ethanol, 1-propanol, 2-propanol, 1-butanol, 2-butanol, acetonitrile, tetrahydrofurane, acetone, formamide, dioxane and mixtures thereof with each other and/or with water, preferably methanol.

In the present method the mobile phase is preferably selected among methanol, ethanol, 1-propanol, 2-propanol, 1-butanol, 2-butanol, acetonitrile, tetrahydrofurane, formamide, dioxane and mixtures obtained by mixing them with each other and/or with water, optionally with the addition of a buffer, the supplementary mobile phase being selected among the above mentioned mobile phases, containing, however, preferably more water, and the flush phases are preferably chosen among the same solvents as listed under extraction agents.

The use according to the invention of the present method is for detecting or sorting individual items on the basis of the contents of at least one fat-soluble compound, the contents of the compound being automatically compared to fixed parameters in a model of detection or sorting.

A preferred present use for the detection of meat pieces with boar taint is characterized in analyzing on the same analysis sample for skatole and androstenone, a derivatization of androstenone being performed as stated above, and in using a detecting or sorting model in which both compounds are parts. Mainly such carcasses are then discarded for which an analysis of a fat sample shows an excess of predetermined threshold values of the skatole and/or androstenone content.

Finally, a preferred present use is characterized in that an amount of reference compound for skatole and optionally also for androstenone is added to each sample, the reference compounds being as defined above, in which the amount of reference compounds correspond to the desired threshold values of the individual items, and that the evaluation of the exceeding of said threshold values for skatole content and optionally for androstenone content is performed by comparison of analysis values, such as peak height or peak area of said compound(s), with peak height or peak area of the reference compoud or reference compounds.

The invention has above been particularly described with reference to fat-soluble compounds related to male pig smell but it may also be used for analyzing the contents of fat-soluble active agents in fatty pharmaceutical preparations or for the determination of residual concentrations of fat-soluble tracers, such as pesticides or antibiotics, in biological materials.

The invention will now be explained in detail with reference to the drawings, in which

Fig. 1 is a schematical view of the analysis equipments in three positions for use in the method according to the invention,

Fig. 2 shows the positions in a second embodiment of the apparatus,

Fig. 3 is a chromatogram showing separation of indole, skatole and derivatized androstenone, and

Fig. 4 illustrates the selectivity in the chromatographic system in three embodiments of the method according to the invention.

In a slaughterhouse fat samples are taken from the carcasses on the slaughter line or in the cold storage. The individual samples are distinctly labelled to refer back each to their individual carcass. The samples are conveyed to the slaughterhouse laboratory in which an assistant chemist prepares the samples for the HPLC analysis:

A predetermined amount of the sample is carefully weighed out, e.g. 0.5 g fat tissue. The sample is put into a glass and a specific amount of a solvent for the fat-soluble compounds to be determined, e.g. 2 ml is added. The solvent dissolves or opens for instance also the constituents in which the compounds are bonded. If desired, a specific amount may be added, e.g. 0.02 to 1.00 ppm of a reference compound detected by the later HPLC analysis and used as an internal standard to check the results of the examined compound(s).

The glass is placed in a homogenizer in which the fat tissue is finely chopped, thereby facilitating the extraction of the compounds by means of the solvent.

The extract is then subjected to a purification step for removing at least a large portion of the undissolved material, the glass being centrifuged in an ordinary laboratory centrifuge optinally cooled.

The assistant chemist then transfers the extract to an autosampler glass and places it in the autosampler magazine together with other glases containing extracts from other fat samples that have been treated in the same manner.

If one of the compounds searched for is to be derivatizised before being detected by the HPLC analysis the assistant chemist may add a predetermined amount of derivatization agent and optionally a catalyst to each glass in the magazine.

When a sufficient amount of glasses with extract have been collected in the autosampler magazine, the assistant chemist may start the analysis equipments which automatically analyze the extracts for the contents of the constituents searched for, and the results are afterwards registered and stored in a permanent or transient medium. This may for instance be started after the day's work so that the results are available the next morning.

HPLC equipments are used which comprise an autosampler, a column thermostat, two pumps, a unit with two six-port switching valves and pipe connections, a fluorescence detector and computer system and software for controlling the HPLC equipments and collection and processing of data. The above mentioned components are standard articles on the market.

A precolumn in a precolumn holder and a separation column are further used. They are also standard articles. The internal column diameter is generally 1 to 10 mm, preferably 4 mm. The length of the precolumn is 1 to 25 mm, preferably 4 mm, and the length of the separation column which must be adapted to the particle size and the type of the column contents, the desired approximate time of pass-through, the type of the fat-soluble compounds for analysis and the mobile phase(s) are generally in the range from 20 to 150 mm.

The contents of the columns are a $C_{18}$-material with an average particle size in the range from 2 to 10 $\mu$.

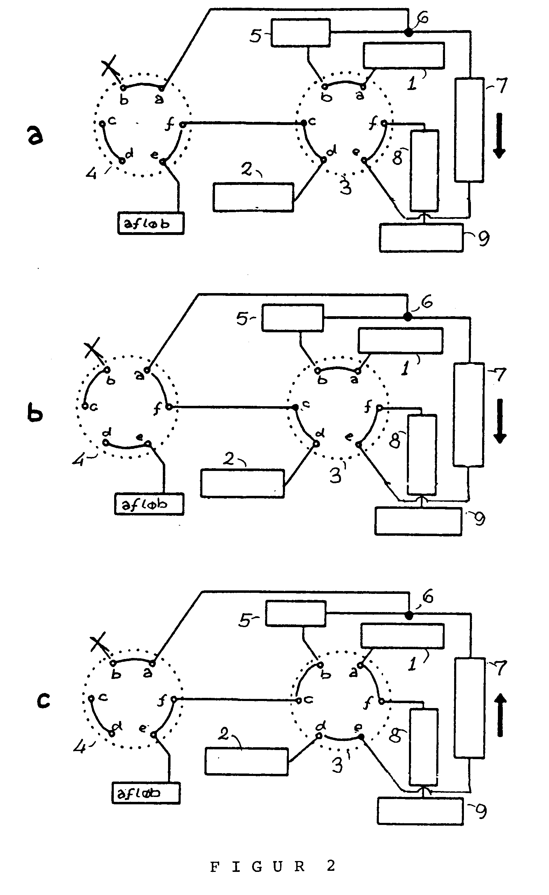

The embodiment illustrated in Fig. 2 makes use of a mixing chamber with three openings. The volume of said chamber is from 1 to 10 $\mu$l.

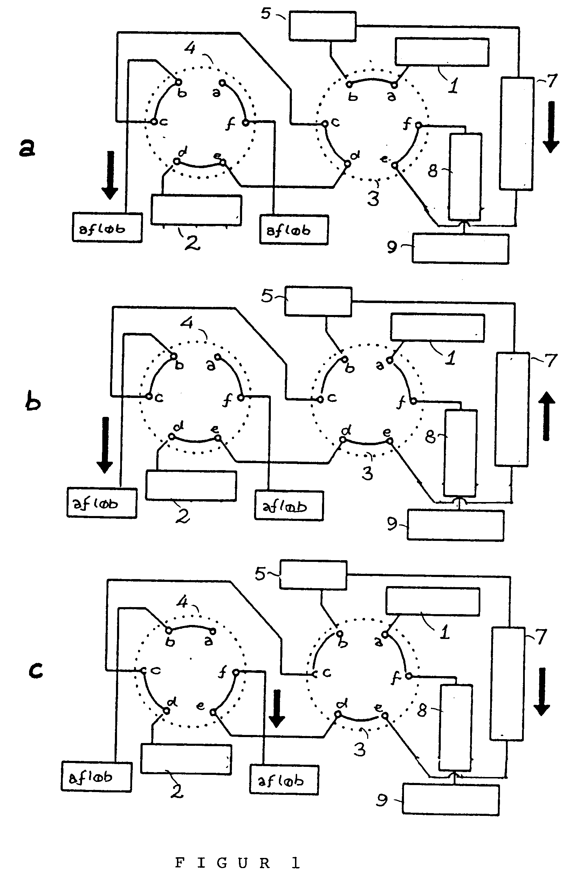

The further analyzing course/cycle is illustrated in Figs 1 and 2.

Fig. 1 shows two pumps 1, 2, two six-port switching valves 3, 4, either of which has six ports 3a to 3f, 4a to 4f. Each valve may occupy two positions, the first one of which designates the connection between the ports 3a to 3b, 3c to 3d and 3e to 3f, and 4a to 4b, 4c to 4d and 4e to 4f, respectively, and the second position designates the connection between the ports 3b to 3c, 3d to 3e and 3f to 3a, and 4b to 4c, 4d to 4e and 4f to 4a, respectively. An autosampler 5, a precolumn 7, a separation column 8 and a fluorescence detector 9 are further shown. The pipe connections are as follows:

from pump 1 to valve port 3a,

from valve port 3b to the autosampler 5,

from the autosampler 5 to the precolumn 7,

from the precolumn 7 to the valve port 3e,

from the valve port 3f to the separation column 8,

from the separation column 8 to the detector 9,

from the pump 2 to the valve port 4d,

from the valve porte 4e to the valve port 3d,

from the valve port 3c to the valve port 4c, and

two connections, from the valve port 4b, and 4f,

respectively, to outlet,

the valve port 4a being moreover not used.

In the situation illustrated in Fig. 1a the valve 3 is in the first position and the valve 4 is in the second position. The pump 1 pumps a phase from a reservoir, not shown, through the autosampler 5, the precolumn 7, the separation column 8 and the detector 9. The pump 2 is in stand-by mode and pumps a very slight amount of the same phase to outlet. Now, a predetermined amount of extract is dosed by the autosampler 5 and this analysis sample is injected automatically through the injection valve of the sampler and is passed through the precolumn 7. Tissue residues will here preferably be caught by the column filter, but fat substances are bonded by the column contents. The fat-soluble compounds searched for rapidly clear the precolumn 7. When they start chromatographing through the separation column 8 the apparatus is automatically switched over into the situation shown in Fig. 1b, the valve 3 being switched into the second position. The pump 1 will now, and moreover also in the situa-tion described below in connection with Fig. 1c, pump the mobile phase through the separation column 8 and the detector 9; the fat-soluble compounds chromatograph at the same time and may gradually be registered by the detector 9 as isolated peaks. If desired, it is possible to change, e.g. via a gradient, from a mobile phase to another (or even more) mobile phases and/or increase the flow rate if a more rapid pass-through is desired of later fat-soluble compounds.

In the situation illustrated in Fig, 1b pump 2 pumps a flush agent from a reservoir, not shown, rearwards through the precolumn 7 and the autosampler 5. The flush agent is chosen so that fat is rapidly released by the contents of the precolumn, fat and tissue residue are flushed out of the system and flow to outlet. If desired, it is possible to change flush agent during the flushing in order to obtain an improved purification of the precolumn 7 etc. To remove flush agent from the system, pump 2 should then be switched to use the first mobile phase.

Valve 4 is then switched to the second position and now the situation illustrated in Fig. 1c prevails, in which the first mobile phase pumped by pump 2 flows forward through the autosampler 5 and the precolumn 7. The valves are then turned and pump 2 is choked to the stand-by mode for reestablishing the situation shown in Fig. 1a and a new analysis may be carried out.

The mobile phase should be selected among such phases capable of causing the fat-soluble compounds to chromatograph through the separation column 8 for a time of e.g. approximately 1 to 10 minutes.

The flush agents as used must have a high resolving power on fat substances bonded by the precolumn 7.

The further analysis course when using an apparatus with mixing chamber appears from Fig. 2. In view of the fact that reference numerals 1 to 5 and 7 to 9 have the same significance as in Fig. 1, a mixing chamber 6 with three openings inserted between the autosampler 5 and the precolumn 7 appears further from Fig. 2. The pipe connections are as follows:

from pump 1 to the valve port 3a,

from the valve port 3b to the autosampler 5,

from the autosampler 5 to the mixing chamber 6,
from the mixing chamber 6 to the precolumn 7,
from the precolumn 7 to the valve port 3e,
from the valve port 3f to the separation column 8,
from the separation column 8 to the detector 9,
from the pump 2 to the valve port 3d,
from the valve port 3c to the valve port 4f, and
from the valve port 4a to the mixing chamber 6,
valve ports 4c, 4d being not used, and valve port 4b being blocked.

In the situation illustrated in Fig. 2a both valves 3, 4 are in the first position, and pump 1 pumps the mobile phase through the autosampler 5, the mixing chamber 6 (whose third opening is blocked), the precolumn 7, the separation column 8 and the detector 9. Pump 2 pumps a further mobile phase to outlet. A predetermined amount of extract is now dosed by the autosampler 5 and is injected into the mobile phase. At the moment when the extract as a "package" in the mobile phase passes the mixing chamber 6 an automatic device, not shown, ensures that the valve 4 is switched to the second position, thereby entailing that pump 2 pumps the supplementary mobile phase acting as a diluent into the mixing chamber 6 via the third opening. This situation appears from Fig. 2b. When the sample has passed, the valves are switched to the situation in Fig. 2c and the further course is in principle as mentioned above in connection with Fig. 1, Fig. 2c thus shows the situation in which pump 1 pumps mobile phase through the separation column 8 and the detector 9 during continued chromatography while pump 2 rearwards flows flush agent through the precolumn 7, the mixing chamber 6 and the autosampler 5. When these components have been equilibrated after completed flushing by means of the mobile phase the situation has been restored and a new analysis may be performed.

As in the embodiment of the present method illustrated in Fig. 1, various mobile phases/flush agents may be used.

EXPERIMENT 1

Appropriate Extraction Agents

The extraction agent employed should preferably have a smaller density that the fat substance contained in the samples so that the extract may easily be separated from solid substances and undissolved fat by centrifugation. Moreover, it must be capable of extracting the substances searched for in a reliable manner. This experiment examines the suitabulity of various solvents.

Fat samples with a skatole content of 0.06 ppm were spiked with 0.2 ppm skatole and 0.2 ppm indole, and for internal standardization further with 0.2 ppm 2-methyl indole. The samples were then extracted, as described in detail in Example 1, by a series of solvents. The extracts were then analyzed in the above described manner for skatole and indole by using 2-methyl indole as the internal standard.

The analysis results showed that mixtures of methanol and 2-propanol in a mixture ratio in the range 25:75-75:25 and mixtures of methanol and acetone in a mixture ratio in the range of 90:10-50:50 were reasonably applicable whereas pure methanol, pure acetonitrile and mixtures thereof in the range 75:25-25:75 were excellently applicable.

Due to work environmental problems with acetonitrile, methanol was preferred as the extraction agent in the following.

**EXPERIMENT 2**

Determination of indole, skatole and androstenone in the same analysis sample

For the evaluation of the possibility of chromatographically separating and determining indole, skatole and derivatizised androstenone a fat sample was extracted by methanol (for details see Example 1), effecting spiking with 0.2 ppm indole, 0.2 ppm skatole and 2 ppm androstenone. After centrifugation 100 $\mu$l 1% TCA (trichloro acetic acid) (w/v in methanol), 100 $\mu$l 2% dansylhydrazine (v/v in methanol) and 100 $\mu$l methanol, were added to 700 $\mu$l of said methanol extract, stored for 30 minutes at 60 °C, following which an analysis was performed on a HPLC system comprising a precolumn and a separation column, but no back flush system.
The mobile phases used were as follows:
a: Acetonitrile/water (900:100 v/v), and

b: Acetonitrile/water (100:900 v/v).
Flow was 1.2 ml/min up to 4 min, afterwards 1.5 ml/min, and
up to 2 min 45% a/55% b was used
from 2 to 3 min this was gradually changed to 100% which was maintained for 8 min,
from 8 to 8.5 min this was gradually changed back to the above and was maintained throughout the experiment (approximately 12½ min.)
the detection was up to 6 min at 280/340 nm and hence at 365/520 nm (excitation/emission).

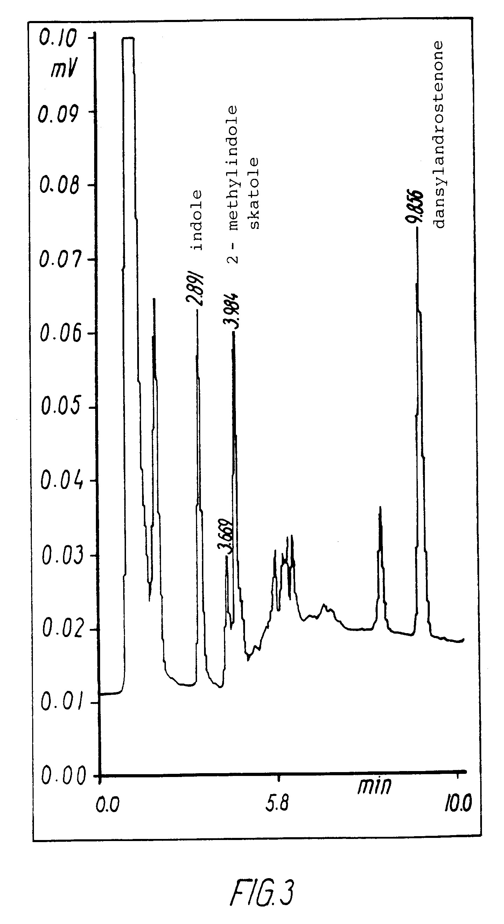

Fig. 3 shows clearly separated peaks for indole and skatole at about 3½ min and about 4½ min, resepctively, a peak for 2-methylindole appears just before skatole. Finally a sharp peak for dansyl androstenone appears at about 9½ min.

The experiment shows that the determination of indole, skatole, the reference compound 2-methyl indole and derivatized (here by dansyl) androstenone may be performed in the same course of analysis and, accordingly, such a determination can be performed by the method according to the invention, meaning that a very safe detection of carcasses with boar taint may be effected, since skatole as well as androstenone and, if desired, also indole may be involved in the determination. This does not require considerable costs for new analyses or other equipments.

## EXAMPLE 1

This Example illustrates the embodiment shown in Fig. 1.

Use is made of a fluorescence detector with excitation at 285 nm and emission at 340 nm and a computer with appropriate software and interface for control .

Precolumn and separation column are as in Experiment 2, the column being thermostated at 40°C.

The mobile phase is acetonitrile/water/0.2 M potassium phosphate buffer pH 3.0 (1000:875: 125 v/v/v), and

the flush phases are acetone/water (950:50 v/v) and acetonitrile/water (950:50 v/v).

### Preparation of Extract

An amount of exactly 0.500 g of a fat sample was weighed out and placed in a 16 mm centrifuge glass, and 2.00 ml of a solution (0.05 ppm) of 2-methylindole in methanol was added.

The sample was then carefully comminuted by means of a homogenizer, the homogenate was placed on an ultrasonic bath for 5 min, cooled to about 15°C, the glass was centrifuged in a centrifuge for 5 min at 15°C and the supernatant was finally transferred to an autosampler glass for analysis.

### Analysis

During the analysis pump 1 was constantly working at 1.2 ml/min. The time periods stated below run from the start of the analysis test. The situation illustrated in Fig. 1a lasted 0.3 min, change-over was hereafter effected to the situation illustrated in Fig. 1b and for 0.9 to 1.7 min the precolumn and autosampler were washed with 1.5 ml/min acetonitrile/water and then, up to 2.9 min with acetone/water. The rearwards flushing continued with the eluate up to 3.5 min, and after 4 min the flushing was directed forwards (Fig. 1c) with 1.2 ml/min of eluent. The analysis lasted 4.8 min in total.

The autosampler dosed in different experiments samples of 10 to 30 $\mu$l, and it turned out that the resolution in the registered chromatogram decreased at increasing size of sample, indicating that the methanol which as a package surrounds the analysis sample passing the chromatographic system to a certain degree will interfere with the chromatography.

The method was validated on samples spiked with 0.04, 0.2 and 0.4 ppm skatole and indole, respectively. The results appear from the following Table.

## TABLE 1

### Validation of the Method According to the Invention
### (perfomed as in Fig. 1)

| Level | Fat piece 1 | | Fat piece 2 | | Fat piace 3 | |
|---|---|---|---|---|---|---|
| | indole | skatole | indole | skatole | indole | skatole |
| 0,04 ppm | | | | | | |
| Found (n=5) | 0.047 | 0.046 | 0.047 | 0.040 | 0.048 | 0.036 |
| st.dev. | 0.003 | 0.003 | 0.006 | 0.009 | 0.003 | 0.010 |
| Recovery% | 118.0 | 117.0 | 118.4 | 100.6 | 121.0 | 90.6 |
| 0.2 ppm | | | | | | |
| Found (n=5) | 0.222 | 0.206 | 0.223 | 0.203 | 0.229 | 0.196 |
| st.dev. | 0.007 | 0.006 | 0.006 | 0.008 | 0.008 | 0.018 |
| c.v.% (n=5) day to day | 3.2 | 3.0 | 3.1 | 4.1 | 3.8 | 9.2 |
| c.v.% (n=7) same day | 5.4 | 2.7 | 7.9 | 5.5 | 7.0 | 3.9 |
| Recovery% | 111.2 | 103.1 | 111.5 | 101.7 | 114.6 | 98.2 |
| 0.4 ppm | | | | | | |
| Found (n=5) | 0.436 | 0.403 | 0.433 | 0.398 | 0.450 | 0.395 |
| st.dev. | 0.021 | 0.014 | 0.024 | 0.009 | 0.019 | 0.016 |
| Recovery% | 109.1 | 100.9 | 108.4 | 99.5 | 112.6 | 98.8 |

c.v. = coefficient of variation

st.dev.= standard deviation

Moreover, the selectivity in the chromatographic system was examined, the following having been analyzed by the method concerned:

A:   a 0.2 ppm (of skatole and indole) standard solution,

B:   a fat sample to which was added 0.2 ppm 2-methylindole, and

C:   the same sample spiked with 0.2 ppm skatole and indole.

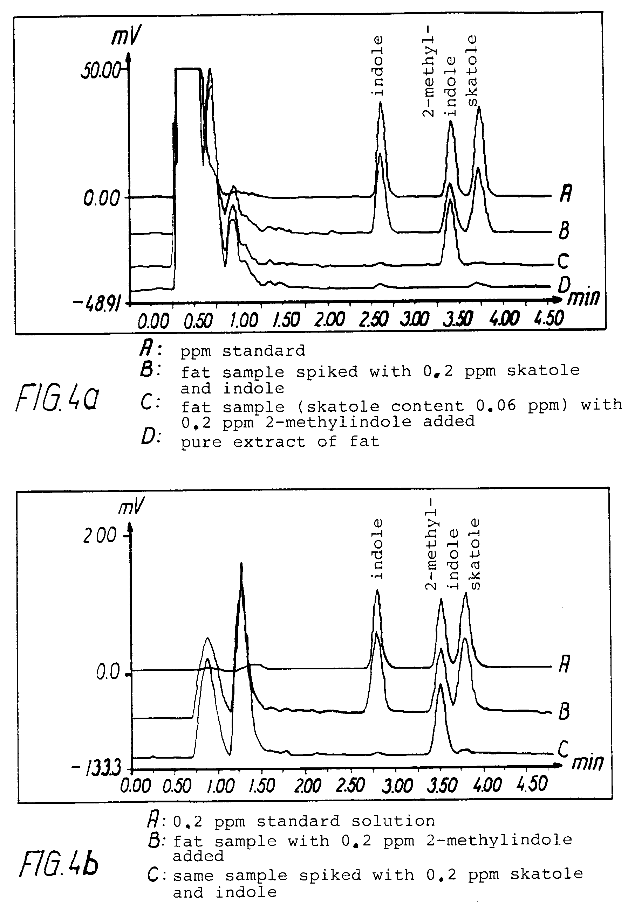

The results appear from Fig. 4a.

Table 1 shows very constant percentages of recovery of skatole and indole, respectively, at 0.2 and 0.4 ppm, but it will also be seen that the standard deviation is sufficiently small to obtain a precision that is completely satisfactory for the detection of male pig smell.

If the present method is used in the sorting-out of slaughtered, uncastrated male pigs a determined amount of 2-methylindole corresponding to the sorting-out limit may be added to all analysis samples. A criterion of sorting out such a carcass may then consist in that the skatole peak is higher than the peak of 2-methylindole.

**EXAMPLE 2**

This example illustrates the embodiment shown in Fig. 2.

The HPLC system includes a 3,1 $\mu$l mixing chamber.

The mobile phase was as stated above. For the dilution of the analysis use was made of a supplementary mobile phase acetonitrile/water (50:950 v/v), and acetonitrile/water (950:50 v/v) was used for the flushing.

The rate of the pump 1 was immediately after starting the analysis raised from 0.7 ml/min to 1.2 ml/min and at the end of the analyzing (at 4.9 min) again lowered to 0.7 ml/min.

The pump 2 pumped at 0 to 0.1 min 0.7 ml/min diluent into the mixing chamber, for a period of 0.9 to 3.0 min. the precolumn, the mixing chamber and the autosampler were backwards flushed with flush agent and for a period of 3.2 to 4.6 min with the eluent.

The resolution was considerably improved by this method.

The selectivity in the chromatographic system was examined as in Example 1 and the results appear in graphic form from Fig. 4b.

**EXAMPLE 3**

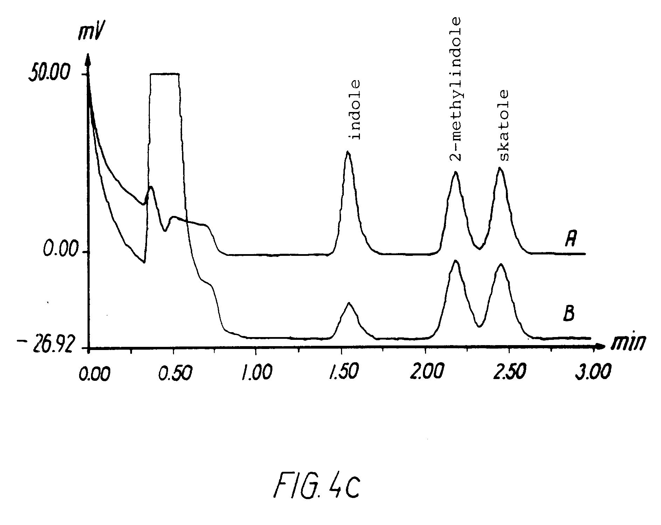

Example 1 was repeated with a second separation column with a faster pass-through. The obtained selectivity is shown graphically on Fig. 4c. The obtained selectivity appears graphically from Fig. 4c. In spite of the faster pass-through (appr. 2½ min) a clear separation of indole, skatole and 2-methylindole is seen.

**EXAMPLE 4**

This example illustrates chromatographic separation of indole, skatole and derivatizised androstenone, the internal standards as used being 2-methylindole (for indole and skatole) and derivatizised androstanone (for androstenone). Only the deviations from the test conditions in Example 1 are described in the following.

Use was made of a second mobile phase containing acetonitrile/tetrahydrofurane/potassium phosphate buffer pH 6/acetic acid (34%:31%:33%:2% v/v).

As internal standards 0.2 $\mu$g/g 2-methylindole and 0.5 $\mu$g/g androstanone were added to the extract and a derivatization was effected to obtain strongly fluorescent derivatives of androstenone and androstanone, respectively, 40 $\mu$l 2% dansyl hydrazine in methanol and as catalyst for the derivatization 40 $\mu$l 25% boron trifluoride in methanol were added to 300 $\mu$l methanol extract. Under these conditions the derivatization takes 3 min at ambient temperature.

During the first part of the analysis the same mobile phase was employed as in Example 1 and the fluorescence detection was at 285/340 nm. After 3 minutes the mobile phase was changed without gradient to the above mentioned second phase and after 5 minutes the fluorescence detection was changed to a wavelength set of 346/521 nm.

Indole, 2-methylindole and skatole were as previously shown as 3 distinct peaks (after 2 1/4, 3 and 3 1/4 min, respectively) and, in addition, distinct peaks for androstenone and androstanone derivatized by dansyl were observed after a period of appr. 8 1/4 and 9 1/4, respectively, of the analysis.

In a series of experiments as described above recovery percentages were found as described above for indole and skatole, whereas the recovery for androstenone was found to 125%. Even though this value differed rather much from the ideal value of 100% it was reproducible, and the experiment thus shows that indole, skatole and androstenone may be determined simultaneously in fat extracts in a single analyzing cycle and an improved security is thus obtained if such an analysis is used as a help in sorting out such carcasses from uncastrated male pigs that are suspected of emitting boar taint when being cooked.

**EXAMPLE 5**

This example illustrates the determination of skatole in small amounts in blood samples from pigs.

Blood samples were sampled in connection with sticking the pigs and 0.50 ml serum taken by pipette from such a sample was inserted into a centrifuge glass, 1.00 ml acetonitrile was added, followed by the steps: mixing, standing in deep-freezer for 15 min and centrifuging for 5 min at a high rate to separate plasma proteins.

For the HPLC analysis of analysis samples (50 $\mu$l) therefrom the analyzer included besides the injection device, columns, pumps, valves and fluorescence detector (set at 285/340 nm Ex/Em) also a mixing chamber positioned in front of the separation column.

The mobile phase during the chromatographic analysis was acetonitrile/0.2 M phosphate buffer pH 3.0/water (800:125:1075 v/v/v). The rate of flow thereof for the first 0.4 min of an analysis was 0.6 ml/min and then 1.5 ml/min. During the first of said periods of time an amount of 0.8 ml/min of a supplementary mobile phase consisting of 0.8 ml % by weight of acetonitrile in water was admixed through the mixing chamber.

Surprisingly, a good separation of the peaks in the registered spectrum was obtained and it was possible to reproducibly determine skatole in amounts down to 0.005 ppm in analysis samples from blood.

By experiments with different acetonitrile concentrations in the analysis sample (after the mixing chamber) noticeably poorer analysis results were observed if acetonitrile concentrations exceeded 45 vol%.

The lifetime of the separation column is held to be extremely good when using the method according to the invention, a column being held to be capable of treating 2000 to 5000 injections. The less expensive precolumn also has a longer lifetime and may be replaced by routine after e.g. 200 to 300 injections.

**Claims**

1.  A method of the examination of individual items for the contents of at least one fat-soluble compound, such as a compound connected with male pig smell, said method comprising as regards each individual item the repetition of the following series of operations:

    taking a sample from said individual item,

    treating the sample with a solvent for the fat-soluble compound,

    separation of solid particles from the obtained solution, and

    carrying out an HPLC analysis on an analysis sample of the purified solution, thereby causing the sample to be injected into a mobile phase passed through a separation column provided with a detector the signals of which are automatically converted to an expression of the contents of the compound, characterized in that the analysis sample in the mobile phase is passed through a precolumn positioned in front of the separation column and that the precolumn is subsequently washed separately for a predetermined period of time.

2.  A method according to claim 1, characterized in that the washing of the precolumn is effected while reversing the flow direction therein.

3.  A method according to any of claims 1 to 2, characterized in that the analysis sample in the mobile phase is passed further through the separation column while washing the precolumn.

4.  A method according to any of claims 1 to 3, characterized in that the sample is a fat sample from a carcass and that the precolumn is a precolumn delaying fatty substances.

5.  A method according to any of claims 1 to 4, characterized by the following steps:

    that the mobile phase is passed through the precolumn and the separation column,

    that an analysis sample representing a single individual is automatically removed from a magazine of separately produced and stored extracts,

    that a predetermined amount of the sample is injected into the mobile phase and passed along with it through the precolumn,

    that the analysis sample in the mobile phase is passed further on in the separation column while reversing the direction of flow in the precolumn and washing the precolumn with one or more, preferably fat-soluble flush phases and - if desired - finally with the same agent as the mobile phase, and

    that the flow direction in the precolumn is subsequently reversed again.

**6.** A method of the examination of individual items for the contents of at least one fat-soluble compound, such as a compound connected with male pig smell, said method comprising as regards each individual item the repetition of the following series of operations:

taking a sample from said individual item,

treating the sample with a solvent for the fat-soluble compound,

separation of solid particles from the obtained solution, and

carrying out an HPLC analysis on an analysis sample of the purified solution, thereby causing the sample to be injected into a mobile phase passed through a separation column provided with a detector the signals of which are automatically converted to an expression of the contents of the compound, characterized by adding, for the determination by means of a fluorescence detector of a non-fluorescent, fat-soluble compound, optionally together with one or more other fat-soluble compounds that are fluorescent, to the solution or the analysis sample a derivatization agent making the non-fluorescent compound fluorescent together with a catalyst for the derivatization reaction.

**7.** A method according to claim 6, characterized in that the derivatization agent is a hydrazine compound.

**8.** A method according to claim 7, characterized in that the hydrazine compound is selected among dansyl hydrazine, 4'-hydrazino-2-stilbazole, 4'-hydrazino-2-oxo-1,3-diazole and 2,4-dinitrophenyl hydrazine.

**9.** A method according to claim 6, characterized in that the catalyst is a Lewis acid.

**10.** A method according to claim 9, characterized in that the catalyst is boron trifluoride.

**11.** A method according to one or more of claims 6 to 10, characterized in that the method is moreover a method according to one or more of claims 1 to 5.

**12.** A method of the examination of inidivdual items for the contents of at least one fat-soluble compound, such as a compound connected with male pig smell, said method comprising as regards each individual item the repetition of the following series of operations:

taking a sample from said individual item,

treating the sample with a solvent for the fat-soluble compound,

separation of solid particles from the obtained solution, and

carrying out an HPLC analysis on an analysis sample of the purified solution, thereby causing the sample to be injected into a mobile phase passed through a separation column provided with a detector the signals of which are automatically converted to an expression of the contents of the compound, characterized in that the mobile phase is further passing a mixing chamber positioned in front of the column and while the dosed amount of analysis sample is passing the mixing chamber through an opening therein a further mobile phase is being admixed therein.

**13.** A method according to claim 12, characterized in that the sample is a blood sample from an animal, that acetonitrile in a ratio of 1 to 3 parts by volume of acetonitrile to 1 part by volume of the sample is added to plasma or serum from the sample for the precipitation of plasma proteins prior to removing solid particles, and while the analysis sample is passing the mixing chamber a sufficient amount of the further mobile phase is being admixed to reduce the acetonitrile content of the sample to 45 % by volume or less.

**14.** A method according to any of claims 12 to 13, characterized in that the method is moreover a method as stated in one or more of claims 1 to 11.

**15.** A method according to any of the preceding claims, characterized in that a predetermined amount of one or more reference compound(s) related to the compound or compounds to be determinated by the HPLC analysis is added to the taken sample prior to or during the treatment thereof with a solvent.

**16.** A method according to claim 15, characterized by using a compound selected among 2-, 4-, 5-, 6-or 7-methylindole as reference compound for the determination of skatole, and using androstanone as reference compound for the determination of androstenone.

12

17. A method according to any of the preceding claims, characterized in that in connection with the determination of more compounds by HPLC analysis the mobile phase is changed and/or the flow rate of the mobile phase(s) is/are changed to to a faster passthrough after the analysis sample has been injected into the separation column.

18. Use of the method according to any of the preceding claims for detecting or sorting individual items on the basis of the contents of at least one fat-soluble compound connected with male pig smell, the contents of the compound being automatically compared to fixed parameters in a model of detection or sorting.

19. Use as claimed in claim 18, characterized in analyzing for skatole and androstenone on the same analysis sample injected into the mobile phase, a derivatization of androstenone being effected according to claims 6 to 10, and in using a detecting or sorting model in which both compounds are constituents.

20. Use according to claim 18 or 19, characterized in that an amount of reference compound of skatole and optionally also of androstenone is added to each sample, the reference compounds being as defined in claim 16, in which the amount of reference compound conforms to a desired threshold value for sorting out the individual items, and that the evaluation of the exceeding of said threshold value for skatole content and optionally for androstenone content is performed by comparison of analysis values, such as peak height or peak area of said compound(s) with peak height or peak area of the corresponding reference compoud or reference compounds.

21. Use according to any of claims 18 to 20, characterized in that the individual items are carcasses and that such carcasses are sorted out for which an analysis of a blood or fat sample shows excess of predetermined threshold values for the skatole and/or androstenone content.

22. An apparatus for use in automatically carrying out the method according to one or more of claims 1 to 11 comprising a separation column (8) and pumping means (1, 2) for passing a mobile phase through the column and a device arranged in front of the column for injecting an analysis sample into the mobile phase, characterized in that it includes a precolumn (7) arranged between the means for injecting an analysis sample and the separation column (8), and that valves (3, 4) are provided for coupling the precolumn (7) into a separate wash flow for a predetermined time without changing the direction of flow in the separation column.

23. An apparatus according to claim 22, characterized in that the valves (3, 4) reverse the direction of flow in the precolumn when coupled in a separate wash flow for a predetermined time.

24. An apparatus according to claim 23, characterized in that it includes an autosampler (5) with an injection valve for an analysis sample, two six-port switching valves (3, 4) either of which may occupy two positions, a first pump (1) for pumping a mobile phase from a reservoir therefor through a selected port in one of the six-port switching valves (3, 4), a second pump (2) which according to choice may pump a mobile phase, a supplementary mobile phase and flush phases from reservoirs therefor through a second selected port in one of the six-port switching valves (3, 4), a precolumn (7) delaying fatty substances, a separation column (8), a detector (9) and pipe connections between the parts, whereby the pipe connections are coupled in such a manner that the precolumn (7) in a first combination of valve positions forms part of the following series connection:
    first pump (1), autosampler (5), precolumn (7) with non-inverted flow, separation column (8) and detector (9),
    in a second pump combination of valve positions the precolumn (7) forms part of the second series connection:
    second pump (2), precolumn (7) with reverse flow, autosampler (5) and outlet, and
    in a third combination of valve positions the precolumn (7) forms part of the third series connection:
    second pump (2), autosampler (5), precolumn (7) with non-inverted flow and outlet.

25. An apparatus according to any of claims 22 to 24, appropriate for use in automatically carrying out the method according to claim 12, characterized in that it further comprises a mixing chamber (6) with three openings, the first one of which is connected through pipes with the injection valve of the autosampler (5) and the second one with the precolumn (7), and that the precolumn (7) in a first and second combination of valve positions forms part of the first and second series connection, respectively, according to claim 24, the third opening of the mixing chamber being blocked, and that the precolumn (7) in a third combination of valve positions forms part of the first series connection according to claim 24, the third opening of the mixing chamber (6) being at the same time connected with the second pump (2).

F I G U R 1

F I G U R 2

FIG. 3

FIG.4a

A: ppm standard
B: fat sample spiked with 0.2 ppm skatole and indole
C: fat sample (skatole content 0.06 ppm) with 0.2 ppm 2-methylindole added
D: pure extract of fat

FIG.4b

A: 0.2 ppm standard solution
B: fat sample with 0.2 ppm 2-methylindole added
C: same sample spiked with 0.2 ppm skatole and indole

18

FIG. 4c